# EUROPEAN PATENT APPLICATION

(11) **EP 2 266 595 A2**
(43) Date of publication of application: **29.12.2010**
(21) Application number: 10185311.7
(22) Date of filing: 06.02.2003
(51) Int. Cl.: A61K 38/22, A61P 1/00, A61P 31/04

(54) **Treatment of infections and other disorders**

(30) Priority: 06.02.2002 US 354250 P; 25.10.2002 US 421038 P
(62) Divisional of application: 03808930.6
(71) Applicant: Regenerx Biopharmaceuticals, Inc., Bethesda, MD 20814 (US)
(72) Inventor: Goldstein, Allan L., Washington, DC 20037 (US); Finkelstein, Jack JR., Chevy Chase, MD 20815 (US)
(74) Representative: Klunker . Schmitt-Nilson . Hirsch

(57) **Abstract**

Microbial infections including anthrax infection, and gastrointestinal disorders, are treated or prevented by administration of an actin-sequestering peptide including amino acid sequence LKKTET, such as Thymosin β4, an isoform of Thymosin β4, oxidized Thymosin β4, or Tβ4 sulfoxide.

SEQUENCE LISTING

<110>  RegeneRx Biopharmaceuticals, Inc.

<120>  TREATMENT OF INFECTIONS AND OTHER DISORDERS

<130>  K 77321/7

<150>  US 60/421,038
<151>  2002-10-25

<150>  US 60/354,250
<151>  2002-02-06

<160>  1

<170>  PatentIn version 3.2

<210>  1
<211>  6
<212>  PRT
<213>  Homo sapiens

<400>  1

Leu Lys Lys Thr Glu Thr
1               5

## Description

### BACKGROUND OF THE INVENTION

### CROSS-REFERENCE TO RELATED APPLICATION

The present application claims the benefit of U.S. Provisional Application Serial Nos. 60/421,038, filed October 25, 2002 and 60/354,250, filed February 6, 2002.

### 1. FIELD OF THE INVENTION

The present invention relates to te field of the treatment of microbial infections and other gastrointestinal disorders.

### 2. DESCRIPTION OF THE BACKGROUND ART

Treatment of microbial infections, bacterial, viral and fungal, can be difficult by conventional methods. These infections may include gastrointestinal infections (E. coli., H. pylori, VRE, etc.) abdominal infections (peritonitis, pancreatitis, gall bladder infections, etc.), surgical infections and osteomyelitis (bone infection).

Another example of microbial infection is anthrax. Anthrax is an infectious agent caused by Bacillus anthracis, a gram positive organism. It is primarily a disease of herbivores. Anthrax can effect many different vertebrates including humans. The symptoms of anthrax vary widely depending on the route of infection. Three forms of the disease commonly occur including a cutaneous form, a gastrointestinal form, and a pulmonary (inhalation) from. The pulmonary form of the disease is typically caused by inhalation of anthrax spores. The symptoms systemic anthrax can be mimicked in a number of animal models by the administration of virulence factors (endotoxins) which are responsible for the major pathologies, morbidity and mortality seen with anthrax. Once large amounts of anthrax toxins are produced within the body by bacteria, administration of antibiotics are usually ineffective. Anthrax induced pathologies mimic septic shock and the sudden death seen with other gram positive and gram negative bacterial infections such as multi-organ failure, edema and ARDS. In both animals and humans the anthrax induced pathologies also include marked elevation of TNFa, IL-1β, PAF and a number of other inflammatory cytokines. Also seen in anthrax induced septic shock is over production of reactive oxygen intermediates and an increase in aracidonic acid metabolites such as PGE, and thromboxane β₂ and disruption of the actin cytoskeleton.

A number of approaches have been reported to delay, prevent and/or treat exposure to anthrax. In the prevention area, a human vaccine is available by the effectiveness of the vaccine is unclear. The best treatment currently available is treatment with specific antibiotics such as ciprofloxaxin or doxycyclin. Antibiotics are effective if given at the very early stages of infection and are basically ineffective once the bacteria have had a chance to multiply rapidly producing lethal amounts of the deadly anthrax toxins. Of the three forms of anthrax, the most deadly form is pulmonary (Inhalation) anthrax which has a fatality rate of greater than 75%(even with appropriate antibiotic treatment). Anthrax produces a multi-component toxin that is assembled at the surface of host cells after infection. The lethal action of the anthrax toxins occurs in the cytoplasm of the host cells. The anthrax toxin is only one of many multi-subunit toxins that cause sever illness in humans. A major concern when treating bacterial infections with antibiotics is the appearance of increasing numbers of antibiotic resistant strains. In addition, once the anthrax bacillus has produced large amounts of exotoxins the antibiotics are basically ineffective.

Millions of Americans suffer from other gastrointestinal (GI) disorders such as colitis, ileitis, Crohn's disease, ulcerative colitis, colic, gingivitis, regional enteritis, ulcers, pouchitis, sclerosing, cholangitis, fistulae. The cause of many of these diseases is not known. However, they may have genetic roots or result from exposure to certain chemicals, pathogens, immune dysfunction, or foods during one's lifetime, or result from the normal aging of the human body. GI disorders occur in both men and women and can be acute or chromic, debilitating and lifethreatening, and may occur anywhere within the GI tract, including but not limited to the mouth, throat, esophagus, stomach, small and large intestines, colon, and anus. People suffering from GI disorders may have a greatly diminished quality of life and suffer premature death.

A large number of therapeutic approaches to treatment have been reported for gastrointestinal disorders and disease, depending upon the location and the nature of the GI pathology. The treatments vary from surgical intervention, to dietary manipulations, to the use of a variety of drugs and biological agents. These agents include antibiotics, anti-virals, antiinflammatory drugs, glucocorticoids, immunosuppressive drugs, monoclonal antibodies, antacids, anti-secretory drugs, anti-spasmodics, as well as a large number of others.

Numerous pharmaceutical, nutriceutical or cosmeceutical formulations have been proposed to treat the damage caused by microbial infections and gastrointestinal disorders.

There remains a need in the art for improved methods and compositions for healing or preventing the damage caused by microbial infections and gastrointestinal disorders.

### SUMMARY OF THE INVENTION

In accordance with the present invention, treatment of infections and gastrointestinal (GI) disorders, comprises administering to a subject in need of such treatment an effective amount of a composition comprising amino acid sequence LKKTET, or a conservative variant thereof.

### DETAILED DESCRIPTION OF THE INVENTION

The present invention is based on a discovery that actin-sequestering peptides such as thymosin β4 (Tβ4) and other actin-sequestering peptides or peptide fragments containing amino acid sequence LKKTET or conservative variants thereof, promote treatment of microbial infections and gastrointestinal disorders. Without being bound to any particular theory, these peptides may have the capacity to promote repair, healing and prevention by having the ability to induce terminal deoxynucleotidyl transferase (a non-template directed DNA polymerase), to decrease the levels of one or more inflammatory cytokines or chemokines, and to act as a chemotactic and/or angiogenic factor for endothelial cells and thus treat damage caused by microbial infections and gastrointestinal disorders.

Thymosin β4 was initially identified as a protein that is up-regulated during endothelial cell migration and differentiation *in vitro.* Thymosin β4 was originally isolated from the thymus and is a 43 amino acid, 4.9 kDa ubiquitous polypeptide identified in a variety of tissues. Several roles have been ascribed to this protein including a role in a endothelial cell differentiation and migration, T cell differentiation, actin sequestration and vascularization.

In accordance with one embodiment, the invention is a method of treatment of damage associated with microbial infections comprising administering to a subject in need of such treatment an effective amount of a composition comprising a microbial infection-inhibiting polypeptide comprising LKKTET, or a conservative variant thereof having microbial infection-inhibiting activity, preferably Thymosin β4, an isoform of Thymosin β4, oxidized Thymosin β4, Thymosin β4 sulfoxide, or an antagonist of Thymosin β4.

Compositions which may be used in accordance with the present invention include Thymosin β4 (Tβ4), Tβ4 isoforms, oxidized Tβ4, Thymosin β4 sulfoxide, polypeptides or any other actin sequestering or bundling proteins having actin binding domains, or peptide fragments comprising or consisting essentially of the amino acid sequence LKKTET or conservative variants thereof, having microbial infection-inhibiting activity. International Application Serial No. PCT/US99/17282, incorporated herein by reference, discloses isoforms of Tβ4 which may be useful in accordance with the present invention as well as amino acid sequence LKKTET and conservative variants thereof having microbial infection-inhibiting activity, which may be utilized with the present invention. International Application Serial No. PCT/GB99/00833 (WO 99/49883), incorporated herein by reference, discloses oxidized Thymosin β4 which may be utilized in accordance with the present invention. Although the present invention is described primarily hereinafter with respect to Tβ4 and Tβ4 isoforms, it is to be understood that the following description is intended to be equally applicable to amino acid sequence LKKTET, peptides and fragments comprising or consisting essentially of LKKTET, conservative variants thereof having microbial infection-inhibiting activity, as well as oxidized

Thymosin β4.

In one embodiment, the invention provides a method for healing damage caused by microbial infection in a subject by contacting an area to be treated with an effective amount of a microbial infection-inhibiting composition which contains Tβ4 or a Tβ4 isoform. The contacting may be topically, systemically or enterally. Examples of topical administration include, for example, contacting the skin with a lotion, salve, gel, cream, paste, spray, suspension, dispersion, hydrogel, ointment, or oil comprising Tβ4, alone or in combination with at least one agent that enhances Tβ4 penetration, or delays or slows release of Tβ4 peptides into the area to be treated. Systemic administration includes, for example, intravenous, intraperitoneal, intramuscular or subcutaneous injections, or inhalation, transdermal or oral administration of a composition containing Tβ4 or a Tβ4 isoform, etc. Enteral administration may include oral or rectal administration. A subject may be a mammal, preferably human.

Tβ4, or its analogues, isoforms or derivatives, may be administered in any effective amount. For example, Tβ4 may be administered in dosages within the range of about 0.1-50 micrograms of Tβ4, more preferably in amounts of about 1-25 micrograms.

A composition in accordance with the present invention can be administered daily, every other day, etc., with a single administration or multiple administrations per day of administration, such as applications 2, 3, 4 or more times per day of administration.

Tβ4 isoforms have been identified and have about 70%, or about 75%, or about 80% or more homology to the known amino acid sequence of Tβ4. Such isoforms include, for example, Tβ4^{ala}, Tβ9, Tβ10, Tβ11, Tβ12, Tβ13, Tβ14 and Tβ15. Similar to Tβ4, the Tβ10 and Tβ15 isoforms have been shown to sequester actin. Tβ4, Tβ10 and Tβ15, as well as these other isoforms share an amino acid sequence, LKKTET, that appears to be involved in mediating actin sequestration or binding. Although not wishing to be bound to any particular theory, the activity of Tβ4 isoforms may be due, in part, to the ability to regulate the polymerization of actin. For example, Tβ4 can modulate actin polymerization in skin (*e*.*g*. β-thymosins appear to depolymerize F-actin by sequestering free G-actin). Tβ4's ability to modulate actin polymerization may therefore be due to all, or in part, its ability to bind to or sequester actin via the LKKTET sequence. Thus, as with Tβ4, other proteins which bind or sequester actin, or modulate actin polymerization, including Tβ4 isoforms having the amino acid sequence LKKTET, are likely to reduce microbial infection alone or in a combination with Tβ4, as set forth herein.

Thus, it is specifically contemplated that known Tβ4 isoforms, such as Tβ4^{ala}, Tβ9, Tβ10, Tβ11, Tβ12, Tβ13, Tβ14 and Tβ15, as well as Tβ4 isoforms not yet identified, will be useful in the methods of the invention. As such Tβ4 isoforms are useful in the methods of the invention, including the methods practiced in a subject. The invention therefore further provides pharmaceutical compositions comprising Tβ4, as well as Tβ4 isoforms Tp4^{ala}, Tβ9, Tβ10, Tβ11, Tβ12, Tβ13, Tβ14 and Tβ15, and a pharmaceutically acceptable carrier.

In addition, other proteins having actin sequestering or binding capability, or that can mobilize actin or modulate actin polymerization, as demonstrated in an appropriate sequestering, binding, mobilization or polymerization assay, or identified by the presence of an amino acid sequence that mediates actin binding, such as LKKTET, for example, can similarly be employed in the methods of the invention. Such proteins include gelsolin, vitamin D binding protein (DBP), profilin, cofilin, adsevertin, propomyosin, fincilin, depactin, Dnasel, vilin, fragmin, severin, capping protein, β-actinin and acumentin, for example. As such methods include those practiced in a subject, the invention further provides pharmaceutical compositions comprising gelsolin, vitamin D binding protein (DBP), profilin, cofilin, depactin, DnaseI, vilin, fragmin, severin, capping protein, β-actinin and acumentin as set forth herein. Thus, the invention includes the use of a microbial infection-inhibiting polypeptide comprising the amino acid sequence LKKTET (which may be within its primary amino acid sequence) and conservative variants thereof.

As used herein, the term "conservative variant" or grammatical variations thereof denotes the replacement of an amino acid residue by another, biologically similar residue. Examples of conservative variations include the replacement of a hydrophobic residue such as isoleucine, valine, leucine or methionine for another, the replacement of a polar residue for another, such as the substitution of arginine for lysine, glutamic for aspartic acids, or glutamine for asparagine, and the like.

Tβ4 has been localized to a number of tissue and cell types and thus, agents which stimulate the production of Tβ4 can be added to or comprise a composition to effect Tβ4 production from a tissue and/or a cell. Such agents include members of the family of growth factors, such as insulin-like growth factor (IGF-1), platelet derived growth factor (PDGF), epidermal growth factor (EGF), transforming growth factor beta (TGF-β), basic fibroblast growth factor (bFGF), thymosin α1 (Tα1) and vascular endothelial growth factor (VEGF). More preferably, the agent is transforming growth factor beta (TGF-β) or other members of the TGF-β superfamily. Tβ4 compositions of the invention may reduce the affects of microbial infection by effectuating growth of the connective tissue through extracellular matrix deposition, cellular migration and vascularization of the skin.

Additionally, other agents may be added to a composition along with Tβ4 or a Tβ4 isoform. Such agents include angiogenic agents, growth factors, agents that direct differentiation of cells, agents that promote migration of cells and agents that stimulate the provision of extracellular matrix material in the skin. For example, and not by way of limitation, Tβ4 or a Tβ4 isoform alone or in combination can be added in combination with any one or more of the following agents: VEGF, KGF, FGF, PDGF, TGFβ, IGF-1, IGF-2, IL-1, prothymosin α and thymosin α1 in an effective amount.

The actual dosage or reagent, formulation or composition that heals damage associated with microbial infection may depend on many factors, including the size and health of a subject. However, persons of ordinary skill in the art can use teachings describing the methods and techniques for determining clinical dosages as disclosed in PCT/US99/17282, *supra*, and the references cited therein, to determine the appropriate dosage to use.

Suitable formulations include Tβ4 or a Tβ4 isoform at a concentration within the range of about 0.001 - 10% by weight, more preferably within the range of about 0.01 - 0.1 % by weight, most preferably about 0.05% by weight.

The therapeutic approaches described herein involve various routes of administration or delivery of reagents or compositions comprising the Tβ4 or other compounds of the invention, including any conventional administration techniques (for example, but not limited to, topical administration, local injection, inhalation, systemic or enteral administration), to a subject. The methods and compositions using or containing Tβ4 or other compounds of the invention may be formulated into pharmaceutical compositions by admixture with pharmaceutically acceptable non-toxic excipients or carriers.

The invention includes use of antibodies which interact with Tβ4 peptide or functional fragments thereof. Antibodies which include pooled monoclonal antibodies with different epitopic specificities, as well as distinct monoclonal antibody preparations are provided. Monoclonal antibodies are made from antigen containing fragments of the protein by methods well known to those skilled in the art as disclosed in PCT/US99/17282, *supra*. The term antibody as used in this invention is meant to include monoclonal and polyclonal antibodies.

In one embodiment, the invention provides a method for treating bacterial infection comprising administering to a subject in need of such treatment, an effective amount of a composition comprising a bacterial infection-inhibiting polypeptide comprising amino acid sequence LKKTET, or a conservative variant thereof having bacterial infection-inhibiting activity.

In another embodiment, the invention provides a method for treating gastrointestinal infection. Common gastrointestinal infections include, but are not limited to Helicobacter pylori (H. pylori), Escherichia coli (E. coli.), vancomycin-resistant Enterococcus faecalis (VRE), and methicillin-resistant Staphylococcus aureus (MRSA). The composition may be delivered systemically by injection, orally, nasally, through suppository or enema, transdermally or any other suitable means.

In another embodiment, the invention provides a method for treating anthrax infection. Damage caused by anthrax infection includes septic shock, sudden death, multi-organ failure, edema, ARDS and inflammatory, degenerative, immunological damage. The composition can be applied alone or in combination with an antibiotic such as ciprofloxocin, doxycyclin, or penicillin. A therapeutically effective amount of the composition is applied to the site or systemically on a periodic basis during a course of therapy to reduce the mortality and morbidity effects of exposure to biological agents such as anthrax or to prevent such effects. The composition may also be delivered systemically by injection, orally, nasally or any other means to reduce the toxicity of pulmonary or gastrointestinal anthrax.

Another aspect of the invention is treatment of other Gastrointestinal disorders. In accordance with one embodiment, the invention is a method of treatment of damage associated with gastrointestinal disorders comprising administering to a subject in need of such treatment an effective amount of a composition comprising a gastrointestinal disorder-inhibiting polypeptide comprising LKKTET, or a conservative variant thereof having gastrointestinal disorder inhibiting activity. This invention is applicable to inflammatory, ulcerative, degenerative, immunological and other injuries to and disorders of the gastrointestinal tract (from the mouth to the anus). These disorders occur due to genetic abnormalities, food intolerance, chemical exposure, aging, and microbial infections.

Gastrointestinal disorders to which the invention is applicable include, but are not limited to, gastrointestinal infections including bacterial, viral and fungal infections, disorders associated with environmental or iatrogenic abrasions, inflammations and other inflammatory disorders, immunological disorders, allergies including food allergies, Crohn's disease, ulcerative colitis, recurrent aphthous stomatitis (recurrent canker sores), ileitis, colic, gingivitis, regional enteritis, ulcers, pouchitis, sclerosing, cholangitis, fistulae and genetic abnormalities. They may result from exposure to certain chemicals, pathogens, immune dysfunction, or foods during one's lifetime, or result from the normal aging of the human body.

In one embodiment, the invention provides a method for healing damage caused by gastrointestinal disorders in a subject by contacting the skin with a gastrointestinal disorder effective amount of a composition which contains Tβ4 or a Tβ4 isoform. The contacting may be topically, enterally or systemically. Examples of topical administration include, for example, contacting the skin with a lotion, salve, gel, cream, paste, spray, suspension, dispersion, hydrogel, ointment, or oil comprising Tβ4, alone or in combination with at least one agent that enhances Tβ4 penetration, or delays or slows release of Tβ4 peptides into the area to be treated. Systemic administration includes, for example, intravenous, intraperitoneal, intramuscular or subcutaneous injections, or inhalation (orally or nasally), transdermal, suppository, enema or oral administration of a composition containing Tβ4 or a Tβ4 isoform, etc. A subject may be a mammal, preferably human.

The invention also is directed to a substance for use in manufacture of a medicament for treatment of microbial infections including anthrax, and gastrointestinal disorders, comprising amino acid sequence LKKTET, or a conservative variant thereof.

### Example

Tβ4 showed antimicrobial activity against Staphylococcus aureus and Escherichia coli at concentrations of 5-20 n mol/ml, and increasingly dose-dependent activity against those microorganisms at concentrations of 50-200 n mol/ml.

Preferred embodiments of the invention are defined in the following paragraphs:
1. A method of treatment of microbial infections comprising administering to a subject in need of such treatment an effective amount of a composition comprising a microbial infection-inhibiting polypeptide comprising amino acid sequence LKKTET, or a conservative variant thereof, having microbial infection-inhibiting activity.
2. The method of para1 wherein said polypeptide comprises Thymosin β4 (Tβ34), an N-terminal variant of Tβ34, a C-terminal variant of Tβ34, an isoform of Tβ4, oxidized Tβ4 or Tβ4 sulfoxide.
3. The method of para1 wherein said composition is administered systemically.
4. The method of para 1 wherein said composition is administered topically.
5. The method of para1 wherein said composition is administered enterally.
6. The method of para1 wherein said microbial infection is a bacterial infection.
7. The method of para1 wherein said microbial infection is an anthrax infection.
8. The method of para1 wherein said microbial infection is a gastrointestinal infection.
9. A method of treatment of gastrointestinal disorders comprising administering to a subject in need of such treatment an effective amount of a composition comprising a gastrointestinal disorder-inhibiting polypeptide comprising amino acid sequence LKKTET, or a conservative variant thereof having gastrointestinal disorder-inhibiting activity.
10. The method of para9 wherein said gastrointestinal disorder is associated with infection, abrasion, inflammation, allergy, immunological disorder, or genetic abnormality.
11. The method of para9 wherein said gastrointestinal disorder is Crohn's disease.
12. The method of para9 wherein said gastrointestinal disorder is ulcerative colitis.
13. The method of para9 wherein said gastrointestinal disorder is recurrent aphthous stomatitis.
14. A substance for use in manufacture of a medicament for treatment of microbial infections, comprising amino acid sequence LKKTET, or a conservative variant thereof, having microbial infection-inhibiting activity.
15. A substance for use in manufacture of a medicament for treatment of gastrointestinal disorders, comprising amino acid sequence LKKTET, or a conservative variant thereof, having gastrointestinal disorder-inhibiting activity.

## Claims

1. A composition for treatment of gastrointestinal disorders and gastrointestinal infections, which comprises a pharmaceutically acceptable carrier and a polypeptide comprising LKKTET or a conservative variant thereof, or which comprises an agent that stimulates production of said polypeptide comprising LKKTET or a conservative variant thereof.

2. The composition of claim 1 wherein said polypeptide comprising LKKTET or a conservative variant thereof is selected from the group consisting of thymosin beta 4 (TB4), an isoform of TB4, oxidized TB4, TB4 sulfoxide, gelsolin, vitamin D binding protein, profilin, cofilin, adseverin, propomyosin, fincilin, depactin, DNase I, vilin, fragmin, severin, capping protein, beta-actinin and acumentin.

3. The composition of claim 2 wherein said isoform of TB4 is selected from the group consisting of TB4^{ala}, TB9, TB10, TB11, TB12, TB13, TB14 and TB15.

4. The composition of claim 1 wherein said agent that stimulates production of said polypeptide comprising LKKTET or a conservative variant thereof is selected from the group consisting of insulin-like growth factor, platelet derived growth factor, epidermal growth factor, transforming growth factor beta, basic fibroblast growth factor, thymosin alpha 1, and vascular endothelial growth factor.

5. The composition of claim 1 which further comprises an angiogenic agent, a growth factor, an agent that directs differentiation of cells, an agent that promotes migration of cells or an agent that stimulates provision of extracellular matrix.

6. The composition of claim 1 which further comprises VEGF, KGF, FGF, PDGF, TGF beta, IGF-1, IGF-2, IL-1, prothymosin or thymosin alpha 1.

7. The composition of claim 1 wherein said gastrointestinal disorder is an inflammatory, ulcerative, allergic, degenerative or immunological disorder, a disorder associated with environmental or iatrogenic abrasions, or due to genetic abnormality, food intolerance, chemical exposure, aging or microbial infection.

8. The composition of claim 1 wherein said gastrointestinal disorder is selected from the group consisting of a bacterial infection, a viral infection, a fungal infection, Crohn's disease, ulcerative colitis, recurrent aphthous stomatitis, ileitis, colic, gingivitis, regional enteritis, ulcers, pouchitis, sclerosing, cholangitis, or a fistula.

9. The composition of claim 1 wherein said gastrointestinal infection is selected from the group consisting of Helicobacter pylori infection, Escherichia coli infection, vancomycin-resistant Enterococcus faecalis infection and methicillin-resistant Staphylococcus aureus infection.

10. The composition of claim 1 which is formulated for delivery by injection, orally, nasally, transdermally, by suppository or by enema.

11. Use of a substance in the manufacture of a medicament for treatment of gastrointestinal disorders and gastrointestinal infections, which comprises a pharmaceutically acceptable carrier and a polypeptide comprising LKKTET or a conservative variant thereof, or which comprises an agent that stimulates production of said polypeptide comprising LKKTET or a conservative variant thereof.

12. The use of claim 11 wherein said polypeptide comprising LKKTET or a conservative variant thereof is selected from the group consisting of thymosin beta 4 (TB4), an isoform of TB4, oxidized TB4, TB4 sulfoxide, gelsolin, vitamin D binding protein, profilin, cofilin, adseverin, propomyosin, fincilin, depactin, DNase I, vilin, fragmin, severin, capping protein, beta-actinin and acumentin.

13. The use of claim 12 wherein said isoform of TB4 is selected from the group consisting of TB4^{ala}, TB9, TB10, TB11, TB12, TB13, TB14 and TB15.

14. The use of claim 11 wherein said agent that stimulates production of said polypeptide comprising LKKTET or a conservative variant thereof is selected from the group consisting of insulin-like growth factor, platelet derived growth factor, epidermal growth factor, transforming growth factor beta, basic fibroblast growth factor, thymosin alpha 1, and vascular endothelial growth factor.

15. The use of claim 11 wherein said substance is used in combination with an angiogenic agent, a growth factor, an agent that directs differentiation of cells, an agent that promotes migration of cells or an agent that stimulates provision of extracellular matrix.

16. The use of claim 11 wherein said substance is used in combination with VEGF, KGF, FGF, PDGF, TGF beta, IGF-1, IGF-2, IL-1, prothymosin or thymosin alpha 1.

17. The use of claim 11 wherein said gastrointestinal disorder is an inflammatory, ulcerative, allergic, degenerative or immunological disorder, a disorder associated with environmental or iatrogenic abrasions, or due to genetic abnormality, food intolerance, chemical exposure, aging or microbial infection.

18. The use of claim 11 wherein said gastrointestinal disorder is selected from the group consisting of a bacterial infection, a viral infection, a fungal infection, Crohn's disease, ulcerative colitis, recurrent aphthous stomatitis, ileitis, colic, gingivitis, regional enteritis, ulcers, pouchitis, sclerosing, cholangitis, or a fistula.

19. The use of claim 11 wherein said gastrointestinal infection is selected from the group consisting of Helicobacter pylori infection, Escherichia coli infection, vancomycin-resistant Enterococcus faecalis infection and methicillin-resistant Staphylococcus aureus infection.

20. The use of claim 11 wherein said substance is formulated for delivery by injection, orally, nasally, transdermally, by suppository or by enema.
